Europäisches Patentamt

European Patent Office

Office européen des brevets

(19)

(11) Publication number: **0 100 904**
**A2**

# (12) EUROPEAN PATENT APPLICATION

(21) Application number: **83106912.5**

(22) Date of filing: **14.07.83**

(51) Int. Cl.³: **C 07 G 7/00**
**A 61 K 39/395**

(30) Priority: **19.07.82 US 399257**
**07.01.83 US 456401**

(43) Date of publication of application:
**22.02.84 Bulletin 84/8**

(84) Designated Contracting States:
**CH DE FR GB LI NL SE**

(71) Applicant: **THE UNITED STATES OF AMERICA**
**represented by the Secretary U.S. Department of**
**Commerce**
**National Technical Information Service Office of**
**Government Inventions and Patents 5285 Port Royal**
**Road**
**Springfield, Virginia 22161(US)**

(72) Inventor: **Neville, David M., Jr.**
**9624 Parkwood Drive**
**Bethesda Maryland 20014(US)**

(72) Inventor: **Youle, Richard J.**
**9629 Connecticut Avenue Apartment 502**
**Washington D.C. 20008(US)**

(74) Representative: **Schüler, Horst, Dr. European Patent**
**Attorney**
**Kaiserstrasse 41**
**D-6000 Frankfurt/Main 1(DE)**

(54) **Improved protocol for the treatment of graft versus host disease.**

(57) This invention relates to a monoclonal antibody known as TA-1 directed against human T-cells and which is covalently linked to the toxin ricin and used to treat human donor bone marrow before the marrow is infused into a human recipient. Additionally, monoclonal antibodies known as TA-1, T101, and UCHT1 covalently linked to separate ricin toxins, are combined in a mixture and used in an improved protocol in the treatment of human donor bone marrow prior to the infusion of the marrow into a human recipient.

EP 0 100 904 A2

0100904

## Description

## Improved Protocol for the Treatment of Graft Versus Host Disease

### Technical Field

This invention relates to a monoclonal antibody known as TA-1 directed against human T-cells and which is covalently linked to the toxin ricin and used to treat human donor bone marrow before the marrow is infused into a human recipient. Additionally, monoclonal antibodies known as TA-1, T101, and UCHT1 covalently linked to separate ricin toxins, are combined in a mixture and used in an improved protocol in the treatment of human donor bone marrow prior to the infusion of the marrow into a human recipient.

### Background Art

Vallera, Daniel A., Richard J. Youle, David M. Neville, Jr., and John M. Kersey, Journal of Experimental Medicine, Vol. 155, pp. 949-954, March 1982.

U.S. Patent 4,359,457 to Neville and Youle teaches the use of Thy 1.2 monoclonal antibody linked to intact ricin toxin as a murine lymphoma cytotoxic reagent.

### Utility Statement

This invention represents an improved protocol in the treatment of graft versus host disease in humans. A single monoclonal antibody identified as TA-1 and, more preferably, a three-part mixture of monoclonal antibodies linked to ricin toxin show superior effectiveness in the elimination of cells which express the T-lymphocyte antigen. These reagents are useful in the treatment of any condition requiring bone marrow transplants.

Description of the Drawings

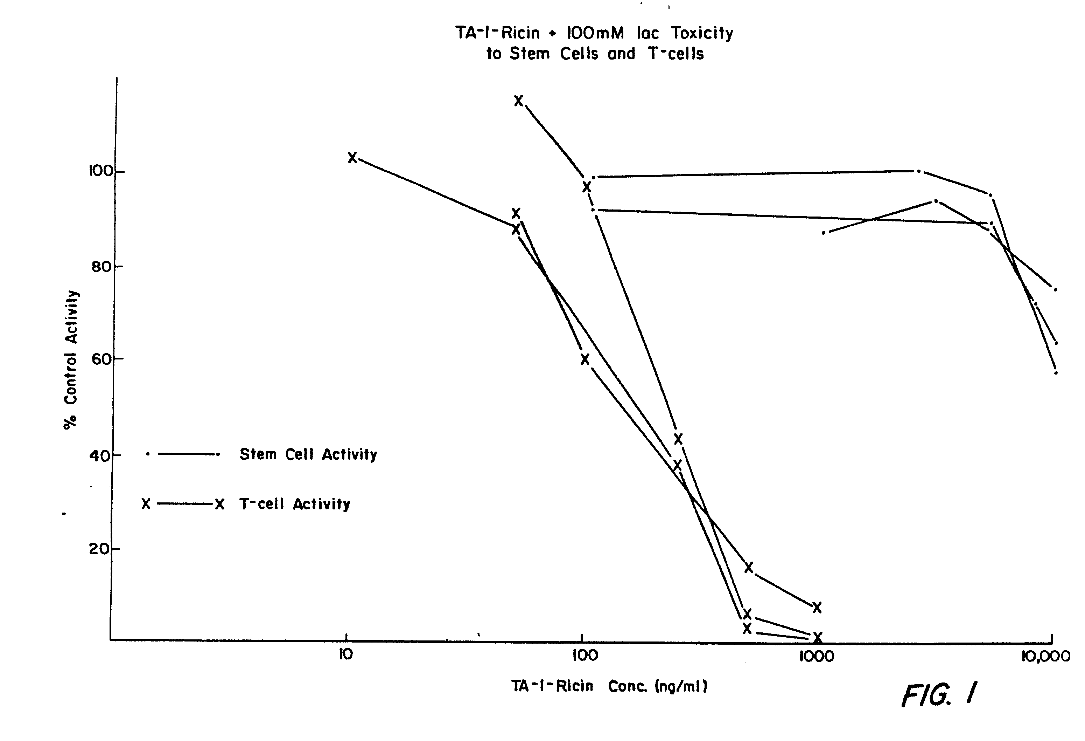

Figure 1 shows the comparison of the toxic effect of TA-1-ricin on human bone marrow stem cells and T-cells.

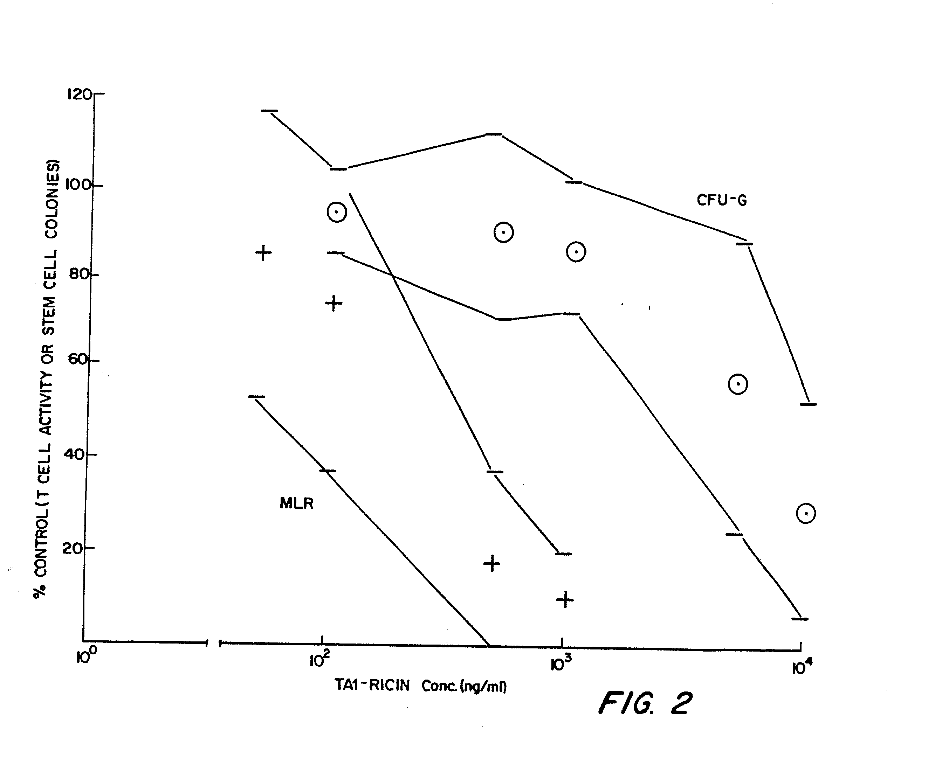

Figure 2 shows human bone marrow and peripheral blood mono-nuclear cells treated with varying concentrations of TA-1-ricin plus lactose. Stem cell activity in terms of colony formation is monitored by the CFU-GEMM assay (colony forming units, granulocyte erythroid monocyte megakaryocyte; see Ash, R.C., et al., Blood, Vol. 58, pages 309-316, 1981). T cell activity is monitored by the MLR assay (mixed lymphocyte reaction; see Bondevik, H., et al., Tissue Antigens, Vol. 4, pages 469-481, 1974). Data points are means of 7-10 separate experiments on separate individuals and hatching indicates ± 1 standard deviation. In spite of large standard deviations, T cell activity is reduced by the reagent at concentrations which have little effect on stem cell activity. However, in some cases even at 1000 ng/ml not all measurable T cell activity is eliminated.

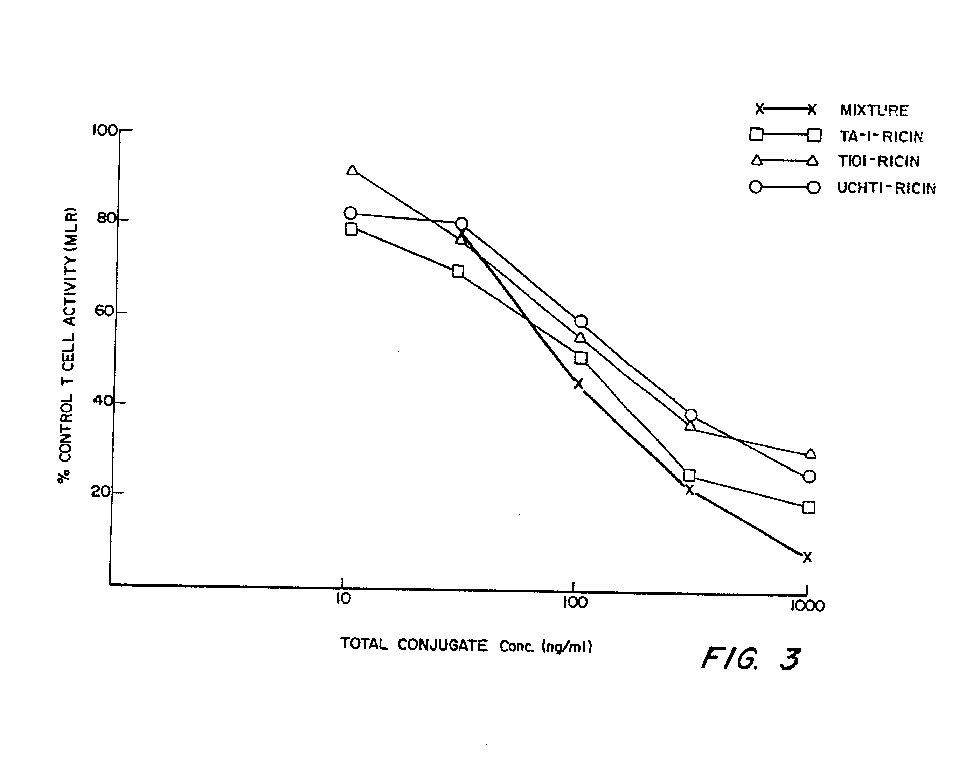

Figure 3 shows human peripheral blood mononuclear cells treated with varying concentrations of the TA-1-ricin, UCHT1-ricin, and T101-ricin and an equal part mixture of all three (shown by the heavy lines). T-cell activity is measured by the MLR assay. Individual and mixture assays are all performed on the same day with the same donor and responder cells. Data are averages of 4 separate experiments. The mixture of conjugates is superior to any of the single conjugates in eliminating T-cell activity.

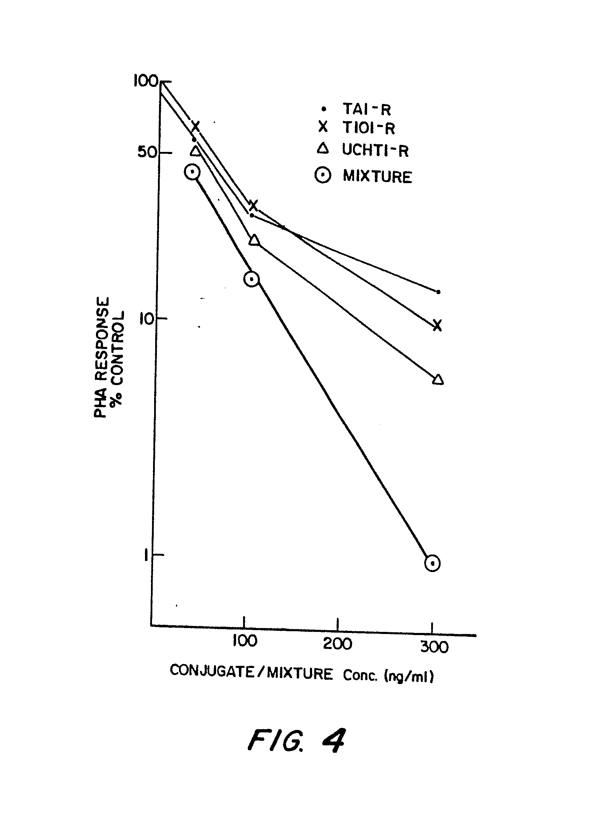

Figure 4 shows human peripheral blood mononuclear cells treated with varying concentrations of the TA-1-ricin, UCHT1-ricin, and T101-ricin and an equal part mixture of all three (shown by the heavy lines). T cell activity is measured by the PHA assay (phytohemagglutinin; see Bernheim, J.L., et al., in Regulatory Mechanism of Lymphocyte Activation, 11th Leucocyte Culture Conference, pages 479-505, 1977). The inhibition of PHA response, plotted on a log scale, deviates from a single hit killing curve for each of the individual conjugates at 300 ng/ml. This indicates that there is a resistant population seen at 300 ng/ml. Likely causes of resistance at high doses are receptor saturation or a subset of cells containing only one or two of the antigenic determinants which TA-1, T101, and UCHT1 are directed against. The mixture, heavy lines, maintains a log linear dose response to 300 ng/ml and provides superior cell killing ability.

General Background

Graft-versus-host disease occurs in patients with aplastic anemia, leukemia, or other diseases who have received bone marrow transplants from non-identical donors. A major impediment to successful bone marrow transplantation is the presence of immunocompetent T lymphocytes in the donor graft which lead to the development of graft-versus-host disease (GVHD). GVHD frequently results in high morbidity and death. T-cells in the donor marrow react against the host and cause the GVHD. The problem in the past has been that elimination of the T-cells in order to prevent the disease has also produced damage to the bone marrow stem cells. Previous attempts at controlling graft-versus-host disease has centered on the use of complement plus antibody to kill the T-cells in bone marrow. However, complement has been shown to be not suitable for clinical use since it is difficult to standardize and is frequently toxic to human bone marrow stem cells. Antibody by itself, that is, without the complement, is not toxic enough to prevent GVHD.

The preliminary experiments, conducted on mice, illustrated that GVHD could be substantially prevented (anti-Thy 1.1) by binding the toxin ricin to monoclonal antibody anti-Thy 1.1 or anti-Thy 1.2. These experiments produced an assortment of moieties which prevented GVHD in mice. Some use anti-Thy 1.1 or anti-Thy 1.2 with intact ricin, others with the A chain or B chain of ricin, and others with ricin A chain followed by separate treatment with ricin B chain. In each case, the problem was the same--transporting a toxic amount of ricin across the cell wall and into the cytosol compartment where protein is made. These experiments. showed (on mice) that an efficient pathway involved the application of ricin linked to anti-Thy 1.1 or anti-Thy 1.2 in a medium containing excess lactose, or ricin A chain linked to anti-Thy 1.1 plus an excess of ricin B chain added separately.

Humans require a different monoclonal antibody directed toward T-cells; however, such antibody must have sufficient affinity and receptor sites to efficiently direct the toxin to the cytosol compartment. The T-cell specific reagents preferred, but not limited thereby, are TA-1, T101, and UCHT1. Like Thy 1.1 and Thy 1.2, TA-1 is a well-known and easily produced monoclonal antibody and is commercially available from Hybritech, LaJolla, California USA (Roitt, I., Essential Immunology, Blackwell Scientific Publications, 1980, Chapters 3 and 4). Human bone marrow is removed from the donor, incubated with TA-1-ricin or a combination of TA-1, T101, and UCHT1, and placed into the human irradiated patient.

The present application teaches human T-cell specific hybrids which selectively kill T-cells in human bone marrow samples without damaging the bone marrow stem cells.

Disclosure of Invention

The present invention teaches the use of singly TA-1 monoclonal antibody as well as a mixture of three different anti-T cell monoclonal antibody ricin conjugates, namely, TA-1, T101, and UCHT1, preferably mixed in equal proportions. (See FIGURE 3 for the results of a comparative study of various concentrations of the individual hybrids). This mixture is more effective in the elimination of T-cell activity while at the same time retaining the same total concentration of antibody ricin as is used in the single hybrid.

In the context of treating human donor bone marrow cells, T-cells in the donor marrow react against the host cells and cause graft versus host disease. T-cell activity, that is, the severity of graft versus host disease following transplantation, is judged in humans by examining the T-cell stimulation by the mixed lymphocyte reaction between donor and recipient cells. Even though the TA1-ricin treatment of the parent application has proven effective, individual variation occurs due to either day-to-day variations of an unknown nature or to patient-to-patient variations (see, for example, the wide $\pm$ 1 standard deviation in FIGURE 1). By mixing together three different anti-T-cell monoclonal antibody ricin conjugates in equal amounts, the ability of the mixture to eliminate T-cell activity is greatly enhanced. It is believed that some of the T-cells carry only one of the determinants necessary to bind the antibody. The mixture of three reagents has proven the most effective treatment possible.

Specific Description

In the synthesis of anti-human-T-cell-ricin hybrid, anti-human-T-cell monoclonal IgG (TA-1) is covalently linked to ricin. TA-1 antibody, commercially available from Hybritech, LaJolla, California, .25 ml at 2.6 mg/ml in 20mM Tris-Cl pH 7.6 is mixed with 25 $\lambda$ of 1 M DTT and incubated at room temperature for 30 min. The DTT is then removed by G25F gel filtration. Ricin D 0.48 ml at 10.6 mg/ml is mixed with 17 $\lambda$ of MBS solution (1.5 mg of m-Maleimidobenzoyl-N-hydroxy-succinimide ester in 0.4 ml dimethyl formamide) and incubated 30 min. at room temperature. The DTT free TA-1 and MBS-ricin are then mixed and incubated 3 hrs. at room temperature. Then 20 $\lambda$ of 0.2M N-ethylmaleimide is added and the hybrid is purified as described in Youle, R.J., and D.M. Neville, Jr., Proc. Natl. Academy Sci., USA, Vol. 77, pp 5483-5486, 1980.

In the synthesis of T101-ricin hybrid, anti-human-T-cell monoclonal T101 is covalently linked to ricin. Antibody T101 is commercially available from Hybritech, La Jolla, California, USA. T101 antibody, .25 ml at 2.6 mg/ml in 20mM Tris-Cl pH 7.6, is mixed with 25 $\lambda$ of 0.1 M DTT and incubated at room temperature for 30 min. The DTT is then removed by G25F gel filtration. Ricin D, 0.24 ml at 10.6 mg/ml, is mixed with 8.0 $\lambda$ of MBS solution (1.5 mg of m-Maleimidobenzoyl-N-hydroxy-succinimide ester in 0.4 ml dimethyl formamide) and incubated 30 min. at room temperature. The DTT free T101 and MBS-ricin are then mixed and incubated 3 hrs. at room temperature. Then 20 $\lambda$ of 0.2M N-ethylmaleimide is added and the hybrid is purified as described in Youle, R.J., and D.M. Neville, Jr., Proc. Natl. Academy Sci., USA, supra.

The third conjugate in the mixture, UCHT1-ricin, is synthesized by the same method used to synthesize TA-1-ricin. UCHT1 monoclonal antibody was made available by Dr. Peter Beverley. A monoclonal antibody, OKT3 with the same specificity and similar affinity as UCHT1, may be substituted for UCHT1 and is commercially available from Ortho Pharmaceuticals.

The protocol used for the actual treatment of human donor bone marrow is as follows: the bone marrow is removed from the human donor, treated in vitro with a mixture of equal parts of TA-1-ricin, T101-ricin, and UCHT1-ricin under excess extracellular lactose conditions, and then infused into the irradiated recipient.

Example 1

The TA-1-ricin hybrid was assayed for its effect on human T-cells and human stem cells. In vitro, the hybrid eliminated T-cells at concentrations 50-fold lower than concentrations to kill stem cells (see Fig. 1).

Procedure:

1. Add 1000 ng TA-1 ricin/ml and lactose 0.1M to bone marrow (BM) cells in 500cc bottle. Then aliquot 40cc BM cells into 50cc conical polystyrene tubes. 40cc PBMs was treated in an identical manner.

2. The 3 sets of tubes were incubated:
   a) BM TA-1 ricin
   b) BM $I^{125}$-TA-1 ricin
   c) PBMs TA-1 ricin

two hours at 37°C with gentle swirling.

3. After incubation, cells were washed treated 2 x with PBS with 1% HA + 15 mM lactose and used as follows:

   a) BM with TA-1 ricin:  washed and pooled 2x in normal saline 1% HSA.  After 2nd wash, pooled BM pellets in 1 tube.  Resuspended in 50cc PBS.  Take aliquot for in vitro experiments (viability studies and CFU-c) and infused the rest.

   b) BM with $I^{125}$-TA-1 ricin:  Checked the amount of ricin to be injected.

   c) PBMs with TA-1 ricin:  Adjusted to proper concentration.

   d) Collected $3 \times 10^8$ nucleated cells/kg body weight.  Approximate yield = $20 \times 10^6$ mononuclear cells/ml.  $20 \times 10^6$ cells/ml x 1000 ml = $20 \times 10^9$ cells in 1 liter.  Recovery from Hetastarch and Ficoll = 25%.

   $.25 \times 20 \times 10^9$ cells = $6 \times 10^9$ cells total

Example 2

Thereafter each hybrid, TA-1-ricin, T101-ricin, and UCHT1-ricin, were assayed for their effect on human T-cells and human stem cells. In vitro, the hybrids eliminated T-cells at concentrations 50-fold lower than concentrations to kill stem cells (see Fig. 2).

Procedure:

1. Add a mixture of 333 ng/ml (final concentration) of each of TA-1-ricin, T101-ricin and UCHT1-ricin to 40 cc of 2% human serum albumin, 100 mM lactose, RPMI medium containing $10^7$/ml density gradient centrifugation purified human nucleated bone marrow cells in 50 cc conical polystyrene tubes.

2. Peripheral blood density gradient purified mononuclear cells are treated in an identical manner and both sets are incubated for 2 hrs at 37° in a $CO_2$ incubator with the pH controlled at 7.35.

3. After incubation, the cells are washed 2 x with PBS containing 1% HA + 15 mM lactose and used as follows:

a. The majority of the bone marrow cells are infused into an irradiated recipient at a dose of 2 x $10^8$ cells/Kg.

b. An aliquot of bone marrow cells are checked for viability in an _in vitro_ clonogenic assay (CFU-GEMM).

c. The peripheral blood mononuclear cells are assayed for elimination of T cell activity (see Fig. 3 and Fig. 4 for this result).

0100904

U/2411

Claims

1. A monoclonal antibody-ricin hybrid named TA-1-ricin used in the treatment of graft versus host disease in humans.

2. A monoclonal antibody-ricin hybrid named T101-ricin used in the treatment of graft versus host disease in humans.

3. A monoclonal antibody-ricin hybrid named UCHT1-ricin used in the treatment of graft versus host disease in humans.

4. A composition for the treatment of graft versus host disease in humans consisting essentially of a mixture of TA-1-ricin, T101-ricin, and UCHT1-ricin.

5. The composition of Claim 4 in which the mixture consists essentially of equal portions of TA-1 ricin, T101-ricin and UCHT1-ricin.

TA-I-Ricin + 100mM lac Toxicity
to Stem Cells and T-cells

FIG. 1

0100904

1/4

FIG. 2

FIG. 3

2/4

0100904

FIG. 4